# EUROPEAN PATENT APPLICATION

(11) **EP 4 563 590 A1**
(43) Date of publication of application: **04.06.2025**
(21) Application number: 23846868.0
(22) Date of filing: 10.07.2023
(51) Int. Cl.: C07K 14/00, A01N 63/50, A61K 38/00, A61P 31/04, C12N 15/70, C12R 1/19

(54) **ANTIBACTERIAL PROTEIN COMPOSITION FOR TREATMENT OF BOVINE MASTITIS**

(30) Priority: 26.07.2022 KR 20220092219
(71) Applicant: Intron Biotechnology, Inc., Seongnam-si, Gyeonggi-do 13202 (KR)
(72) Inventor: YOON, Seong Jun, Seoul 06281 (KR); JUN, Soo Youn, Seoul 06518 (KR); JUNG, Gi Mo, Seoul 08725 (KR); CHOI, Ji Hye, Seongnam-si Gyeonggi-do 13245 (KR); KANG, Sang Hyeon, Seoul 05501 (KR)
(74) Representative: Latscha Schöllhorn Partner AG
(86) International application number: PCT/KR2023/009733
(87) International publication number: WO 2024/025209

(57) **Abstract**

The present invention relates to the antibacterial protein SBL200 with specific antibacterial activity against *Streptococci.* More specifically, the present invention concerns *Streptococcus-*specific antibacterial protein SBL200, and a composition including the Streptococcus-specific antibacterial protein SBL200 as an active ingredient for the treatment of an infection caused by *Streptococci,* wherein the antibacterial protein retains ability to lyse *Streptococci* and possessing the amino acid sequence represented by SEQ ID NO: 1.

## Description

### Technical Field

The present disclosure relates to an antibacterial protein SBL200 and a composition containing the antibacterial protein SBL200 as an active ingredient, the antibacterial protein SBL200 having lytic activity against *Streptococci,* which is one of the causative bacteria of bovine mastitis. More specifically, the present disclosure relates to the antibacterial protein SBL200 specific for *Streptococci,* in which the antibacterial protein SBL200 has an ability to specifically lyse *Streptococci* and contains an amino acid sequence represented by SEQ ID NO: 1, and the present disclosure relates to a composition for treating bovine mastitis, containing the antibacterial protein SBL200 specific for *Streptococci* as an active ingredient.

### Background Art

Bovine mastitis is a disease in which bacteria, which are widely distributed in nature, invade the dairy cow's udder and cause inflammation. Bovine mastitis is the most common disease among dairy cows. Bovine mastitis increases the number of bacteria and somatic cells in milk, resulting in a decrease in milk quality. Bovine mastitis is also a representative dairy cow disease that causes economic losses such as reduced milk flow and treatment costs. Even in the developed country of the United States, the economic loss caused by bovine mastitis is enormous. It is known to cause economic losses of $200 per dairy cow, or $200 million per year.

Bovine mastitis is caused by very complex factors. Factors causing bovine mastitis may be broadly divided into three categories: microorganisms, which are causative bacteria; dairy cows, which are hosts; and environmental factors that affect them all. There are more than 100 types of bacteria known to cause bovine mastitis, and the bacteria are known to cause infections through a wide variety of routes and show diverse disease patterns.

When isolating the main causative agents of bovine mastitis, there are slight differences from case to case, but *Streptococci* account for about 10%. Although the isolation rate cannot be said to be high, it is known that bovine mastitis caused by *Streptococci* is difficult to treat due to its high incidence of antibiotic-resistant bacteria. Therefore, it can be said that there is a need to develop effective drugs to treat bovine mastitis caused by *Streptococci.*

Treatments for bovine mastitis include ointments containing antibiotics such as cephalosporin-based antibiotics and injections containing herbal ingredients such as phytolacca decandra extract. There is great dissatisfaction with the usefulness of these treatments due to insufficient treatment effectiveness and frequent occurrence of resistant bacteria. Considering this, there is a need to develop treatment agents for bovine mastitis utilizing new drugs that provide improved treatment effectiveness and lower incidence of resistance.

### Disclosure

### Technical Problem

Accordingly, as a solution to the problems caused by the use of the conventional antibiotics against the harmful pathogenic bacteria, *Streptococci,* the present inventor(s) seek to provide an antibacterial protein SBL200 which can specifically lyse *Streptococci.* Furthermore, the present inventor(s) further seek to provide a composition, which contains this antibacterial protein as an active ingredient and can be used to treat infections caused by *Streptococci.* Furthermore, the present inventor(s) further seek to provide a method of effectively treating bovine mastitis caused by *Streptococci* using the composition.

Therefore, one objective of the present disclosure is to provide an antibacterial protein SBL200 having an antibacterial activity to specifically lyse *Streptococci* and containing an amino acid sequence represented by SEQ ID NO: 1.

Another objective of the present disclosure is to provide a method of efficiently preparing an antibacterial protein SBL200 having an antibacterial activity to specifically lyse *Streptococci* and containing an amino acid sequence represented by SEQ ID NO: 1. In this regard, *Escherichia coli* M-2 is provided as a production strain of the antibacterial protein SBL200. The production strain *Escherichia coli* M-2 was deposited at the Biological Resource Center of the Korea Research Institute of Bioscience and Biotechnology (KRIBB) on July 12, 2022 (Accession No. KCTC15032BP).

A further objective of the present disclosure is to provide a composition for treating infections caused by *Streptococci,* containing the antibacterial protein SBL200 capable of specifically lysing *Streptococci* as an active ingredient.

In addition, a yet further objective of the present disclosure is to provide a method of treating bovine mastitis caused by *Streptococci,* by using the composition containing the antibacterial protein SBL200 as an active ingredient.

### Technical Solution

To achieve the objectives, the inventor(s) of the present disclosure utilized information on various antibacterial proteins known to have antibacterial activity against various bacterial species, and utilized their knowledge and expertise to the fullest to prepare various antibacterial protein candidates in the form of recombinant proteins and examined lytic activity of the antibacterial protein candidates against *Streptococci.* Thereby, the inventor(s) selected an antibacterial protein with excellent lytic activity and developed a method of efficiently preparing the antibacterial protein, and lastly developed a composition that can be used for treating bovine mastitis caused by *Streptococci* by using the antibacterial protein as an active ingredient. As a result, the inventor(s) completed the present disclosure.

Therefore, according to one aspect of the present disclosure, the present disclosure provides an amino acid sequence of an antibacterial protein SBL200, which may specifically lyse *Streptococci.* Specifically, the amino acid sequence of the antibacterial protein SBL200 corresponds to an amino acid sequence represented by SEQ ID NO: 1. The antibacterial protein SBL200, which may specifically lyse *Streptococci* is made of 291 amino acid residues and has a molecular weight of approximately 32.5 kDa.

It is obvious that the amino acid sequence represented by SEQ ID NO: 1 may be partially modified by those skilled in the art using known techniques. The modifications include partial substitution of the amino acid sequence, partial addition of the amino acid sequence, and partial deletion of the amino acid sequence. However, it is most preferable to apply the amino acid sequence represented by SEQ ID NO: 1 disclosed in the present disclosure.

Additionally, the present disclosure provides a strain *Escherichia coli* M-2, which can be used to produce the antibacterial protein SBL200 containing the amino acid sequence represented by SEQ ID NO: 1. The strain *Escherichia coli M-2* is a strain to produce SBL200, constructed by the present inventor (s) by transforming *E. coli* with a plasmid containing the nucleic acid sequence represented by SEQ ID NO: 2 (6,106 bp).

According to another aspect of the present disclosure, the present disclosure provides a composition containing the antibacterial protein SBL200 as an active ingredient for being effectively used for treating infections caused by *Streptococci,* in which the antibacterial protein SBL200 contains the amino acid sequence represented by SEQ ID NO: 1 and has specific lytic activity against *Streptococci.*

According to the present disclosure, the antibacterial protein SBL200, which is contained in the composition of the present disclosure, contains the amino acid sequence represented by SEQ ID NO: 1 according to the present disclosure, and has specific lytic activity against *Streptococci,* as described above, specifically lyses *Streptococci.* Thus, the antibacterial protein SBL200 is effective in treating various diseases caused by *Streptococci.* Thus, the composition of the present disclosure is developed as an antibiotic, a disinfectant, a sterilizer, and a therapeutic agent to treat various diseases caused by *Streptococci.*

Therefore, according to a further aspect of the present disclosure, the present disclosure provides a method of treating various diseases caused by *Streptococci* by administering the antibacterial protein SBL200 to individuals infected with *Streptococci,* the antibacterial protein SBL2200 containing the amino acid sequence represented by SEQ ID NO: 1 and being specific for *Streptococci.*

The term "disease caused by *Streptococci"* in this specification refers to a disease caused by Streptococcal infections. The disease includes bacteremia, endocarditis, abscess, cavities, pneumonia, meningitis, sinusitis, otitis media, pharyngitis, scarlet fever, pyodermatosis, cellulitis, necrotizing fasciitis, rheumatic fever, glomerulonephritis, neonatal infections (meningitis, pneumonia, and bacteremia), urinary tract infection, amnionitis, endometritis, or wound infections but is not limited thereto.

It does not matter, in this specification, whether *Streptococci* is sensitive to conventional antibiotics or resistant to conventional antibiotics. In other words, it does not matter whether *Streptococci* acquires resistance to conventional antibiotics.

The term "treatment" or "treatment", as used herein, refers to all actions which suppress infections caused by *Streptococci,* and alleviate pathological conditions of diseases caused by *Streptococci.*

To increase efficiency for this purpose, antibacterial substances, which can provide antibacterial activity against other bacterial species, can be added to the composition of the present disclosure.

A pharmaceutically acceptable carrier included in the composition of the present disclosure is one commonly used in preparation. The pharmaceutically acceptable carrier may include any one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but is not limited thereto. In addition to the ingredients, the composition of the present disclosure may further include any one selected from the group consisting of lubricants, wetting agents, sweeteners, flavoring agents, emulsifiers, suspending agents, and preservatives.

The composition of the present disclosure may be administered through oral administration or parenteral administration. In the case of parenteral administration, the composition may be administered using intravenous administration, intraperitoneal administration, intramuscular administration, subcutaneous administration, or local administration. In addition, application or spraying to the affected area may also be used, but the administration method is not limited thereto.

The composition of the present disclosure may be formulated using the pharmaceutically acceptable carrier and/or an excipient by a method which can be easily performed by those skilled in the art to which the present disclosure pertains. Thereby, the composition of the present disclosure may be prepared in unit dosage form or may be prepared by placing the pharmaceutical composition in a multi-dose container. In this case, the formulation of the pharmaceutical composition may be in the form of a solution, suspension, or emulsion in an oil or aqueous medium or may be in the form of an extract, a powder, a granule, a tablet, or a capsule. In the formulation, a dispersant or stabilizer may be further included.

In addition, suitable application, spraying, and dosage of the composition vary depending on factors such as formulation method, administration method, age, weight, sex, severity of disease symptoms, food, administration time, administration route, excretion rate, and reaction sensitivity. Usually, a skilled doctor or veterinarian can easily determine and prescribe an effective dosage for the desired treatment.

The composition of the present disclosure may be developed as an antibiotic, a disinfectant, a sterilizer, and a therapeutic agent.

### Advantageous Effects

A method of treating infections caused by *Streptococci,* by using a composition containing an antibacterial protein SBL200 as an active ingredient, in which the antibacterial protein SBL200 contains an amino acid sequence represented by SEQ ID NO: 1 according to the present disclosure, can also be effective against *Streptococci* which have acquired resistance to conventional antibiotics or antibacterial substances. On the other hand, the antibacterial protein SBL200 of the present disclosure specific for *Streptococci* does not affect normal flora other than *Streptococci,* so it can minimize side effects resulting from the use of the composition containing the antibacterial protein SBL200 as an active ingredient. Meanwhile, conventional antibiotics have the disadvantages of harming beneficial bacteria and causing various side effects.

### Description of Drawings

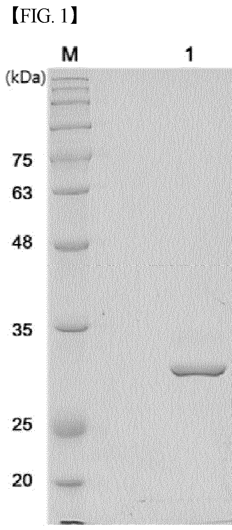

FIG. 1 shows an electrophoresis photograph showing the results of preparing an antibacterial protein SBL200 specific for *Streptococci* in the form of a recombinant protein, the antibacterial protein SBL200 containing an amino acid sequence represented by SEQ ID NO: 1, in which lane M represents a protein size marker, and lane 1 represents a chromatography flow through during secondary purification.

### Best Mode

Hereinafter, the present disclosure will be described in more detail based on examples, but these examples are only illustrative of the present disclosure, and the scope of the present disclosure is not limited to these examples.

### Example 1: Preparation of Streptococci-specific Antibacterial Protein SBL200

Preparation of an antibacterial protein SBL200 specific for *Streptococci,* will be described below. In this example, *Escherichia coli* M-2 was used as a production strain, constructed by the present inventor(s) by transforming *E. coli* with a plasmid containing a nucleic acid sequence represented by SEQ ID NO. 2.

20 µL of *Escherichia coli* M-2 was inoculated into 20 mL of LB medium (Tryptone 10 g/L, yeast extract 5 g/L, and NaCl 10 g/L) supplemented with 50 µg/mL kanamycin. Afterward, the LB medium was cultured with shaking at a temperature of 37°C overnight. The next day, the overnight cultured medium was added at a volume ratio of 1/100 to 1 L of LB medium supplemented with 50 µg/mL kanamycin. Culture was performed at a temperature of 37°C with a stirring speed of 200 rpm. When the cell concentration reached 0.3 to 0.35 based on absorbance at a wavelength of 600 nm, the culture temperature was changed to 19°C. After about 30 minutes, when the cell concentration reached 0.5 based on absorbance at a wavelength of 600 nm, isopropyl β-D-1-thiogalactopyranoside (IPTG) was added so that the final concentration was 0.1 mM. Thereby, expression of an antibacterial protein SBL200 containing an amino acid sequence represented by SEQ ID NO: 1 was induced. Thereafter, an additional 16-hour culture was performed.

After completion of the culture, the cell culture broth was taken and centrifuged at a temperature of 4°C for 10 minutes at 6,000 rpm to recover the cell pellet. The recovered cell pellet was suspended in 10 mL of a buffer (20 mM Tris-HCl, 0.5 M NaCl, and pH 7.5). The cells of the cell suspension prepared in this way were disrupted using sonication. The sonication was performed by applying ultrasonic waves for 3 seconds to break the cells, stopping for 3 seconds. This process was repeated for a total of 15 minutes and in an ice bath.

After the cell disruption, the cell lysate was centrifuged at 13,000 rpm for 20 minutes at a temperature of 4°C, and an obtained supernatant was subjected to a conventional affinity chromatography purification process.

The purification process is briefly explained as follows. In this example, 1 mL of HisTrap^{™} HP (GE Healthcare) was used as an affinity resin. The chromatography was performed after pre-equilibrating a column with a buffer A (20 mM Tris-HCl, 0.5 M NaCl, and pH 7.5). A sample was loaded onto the column. Thereafter, washing was performed by flowing 5 CV (Column Volume) of the buffer A at a flow rate of 1 mL/min. After the washing, the chromatography was performed under the following condition that a concentration gradient from the buffer A to a buffer B (20 mM Tris-HCl, 0.5 M NaCl, 1 M Imidazole, and pH 7.5) was changed from 0% to 100% at a flow rate of 1 mL/min. In this process, elution of the antibacterial protein SBL200 with the desired amino acid sequence represented by SEQ ID NO: 1 was achieved. FIG. 1 shows the results of electrophoresis analysis of the antibacterial protein SBL200 purified using a chromatographic purification process.

Among the obtained purified fractions, those containing high concentrations of the antibacterial protein SBL200 were collected. The collected fractions were dialyzed against a buffer (50 mM Potassium phosphate and pH 7.5) to perform medium exchange. Through this, it was possible to secure the antibacterial protein SBL200 solution with a purity of 90% or more.

Buffer exchange was performed for this concentrated SBL200 solution with a buffer (50 mM Potassium phosphate, 5% (w/v) Sorbitol, 0.1%(w/v) poloxamer 188, and pH 7.5). Thereby, "an SBL200 protein solution" was prepared.

### Example 2: Preparation of Pharmaceutical Composition for Bovine Mastitis Treatment

Using the purified SBL200 solution, an ointment formulation with a composition of "allantoin 2 g/L, sodium benzoate 0.6 g/L, sodium proionate 3 g/L, propylen glycol 50 g/L, SBL200 protein solution 3.6 ml/L" was prepared.

Specifically, the ointment formulation was prepared in the following manner. First, 90 L of sterilized distilled water was added to a preparation tank and then cooled to 30°C or less. Then, the following ingredients-allantoin, sodium propionate, sodium benzoate, and propylene glycol-were sequentially added and dissolved under mixing conditions of 170 rpm. Next, the mixing rpm of the preparation tank was lowered to 100 rpm or less. Then, the SBL200 protein solution prepared in Example 1 was slowly added. The amount of the mixture was adjusted to a final amount using distilled water, and then nitrogen gas injection was stopped. Lastly, the preparation of the product for treatment of bovine mastitis was completed by adjusting the final amount using purified water, and the prepared product was refrigerated.

### Example 3: Investigation of Antibacterial Activity of Antibacterial Protein SBL200

Antibacterial activity of the antibacterial protein SBL200 specific for *Streptococci,* and prepared according to Example 1 was examined. Details of test strains subject to antibacterial activity investigation are shown in Table 1.

**[Table 1]**

| Test Strains Subject to Antibacterial Activity Investigation | | |
|---|---|---|
| Species | Strain | Source |
| *Streptococcus agalactiae* | ATCC 27956 | ATCC |
| | CCARM 4503 | CCARM |
| | CCARM 4512 | |
| *Streptococcus pyogenes* | CCARM 0032 | CCARM |
| | CCARM 0103 | |

| *Staphylococcus aureus* | ATCC 33591 | ATCC |
|---|---|---|
| | CCARM 3090 | CCARM |
| *Acinetobacter baumannii* | CCARM 12199 | CCARM |
| *Clostridioides difficile* | CCARM 0187 | CCARM |
| *Pseudomonas aeruginosa* | CCARM 2239 | CCARM |
| *Klebsiella pneumoniae* | CCARM 10330 | CCARM |
| *Escherichia coli* | CCARM 1G941 | CCARM |
| CCARM: Culture Collection of Antimicrobial Resistance Microbes; | | |
| ATCC: American Type Culture Collection | | |

A turbidity reduction assay and MIC (Minimum Inhibitory Concentration) test were used to investigate the antibacterial activity. The turbidity reduction assay was conducted as follows. Test bacterial strains were suspended in physiological saline so that absorbance of the prepared cell suspension was about 0.7 at a wavelength of 600 nm. Thereafter, 0.1 mL of antibacterial protein SBL200 solution was added (final concentration: 2.5 µM) to 0.9 mL of this suspension. Then, the absorbance was measured at a wavelength of 600 nm for 30 minutes. For preparing a negative control, a buffer (50 mM Potassium phosphate, 5% (w/v) Sorbitol, 0.1%(w/v) poloxamer 188, and pH 7.5) without the antibacterial protein SBL200 was used instead of the antibacterial protein SBL200 solution. An MIC was determined by conducting a routine MIC test in accordance with CLSI guidelines.

As a result, the antibacterial protein SBL200 showed lytic activity only against *Streptococci,* meanwhile, the antibacterial protein SBL200 did not show lytic activity against other test strains. The results are presented in Table 2.

**[Table 2]**

| Antibacterial Activity Investigation Results | | | |
|---|---|---|---|
| Species | Strain | Susceptibility | MIC (µg/ml) |
| *Streptococcus agalactiae* | ATCC 27956 | Susceptible | 6.25 |
| | CCARM 4503 | Susceptible | 6.25 |
| | CCARM 4512 | Susceptible | 6.25 |
| *Streptococcus pyogenes* | CCARM 0032 | Susceptible | 1.56 |
| | CCARM 0103 | Susceptible | 1.56 |
| *Staphylococcus aureus* | ATCC 33591 | Insusceptible | - |
| | CCARM 3090 | Insusceptible | - |
| *Acinetobacter baumannii* | CCARM 12199 | Insusceptible | - |
| *Clostridioides difficile* | CCARM 0187 | Insusceptible | - |
| *Pseudomonas aeruginosa* | CCARM 2239 | Insusceptible | - |
| *Klebsiella pneumoniae* | CCARM 10330 | Insusceptible | - |
| *Escherichia coli* | CCARM 1G941 | Insusceptible | - |

From these results, it was confirmed that the antibacterial activity of the antibacterial protein SBL200 of the present disclosure was significantly specific for *Streptococci.*

From the results, it was confirmed that the antibacterial protein SBL200 specific for *Streptococci* of the present disclosure could lyse *Streptococci,* thereby ultimately contributing to killing *Streptococci.* These antibacterial properties of the antibacterial protein SBL200 showed that the composition containing the antibacterial protein SBL200 specific for *Streptococci* could be used for the purpose of killing *Streptococci* when infections caused by *Streptococci* occurred. In addition, the antibacterial properties of the antibacterial protein SBL200 showed that the composition containing the antibacterial protein SBL200 specific for *Streptococci* could also be used in the same way as conventional antibiotics for the purpose of treating infections caused by *Streptococci.*

### Example 4: Investigation of Antibacterial Activity in Milk of Antibacterial Protein SBL200

The antibacterial activity in milk of the antibacterial protein SBL200 specific for *Streptococci* and prepared according to Example 1 was examined. Milk was inoculated with *S. agalactiae* ATCC 27956 (Bovine mastitis isolate) at a concentration of 1× 10⁵ CFU/mL. The SBL200 protein sample was then added thereto to achieve a final concentration of 50 µg/mL. Next, the mixture was left at room temperature for 30 minutes or 1 hour. 50 µL of a sample was obtained from the mixture and then diluted 100-fold. 100 µL of the diluted sample was spread onto agar plates. Thereafter, the plates were incubated for one day. A bacterial count in the milk was measured by using a conventional plate counting method, which was used to measure the number of colonies formed. The results are shown in FIG. 3. In the results below, the case where the SBL200 protein sample was not added is the control group. In the control group, the bacterial count was measured using the conventional plate counting method in the same manner as the treatment group.

**[Table 3]**

| Results of Bacterial Count Investigation in Milk | | |
|---|---|---|
| Division | Number of Bacteria in 100 µL of Sample | |
| | 30-minute Treatment | 1-Hour Treatment |
| Control Group | 112 | 252 |
| Treatment Group | 0 | 0 |

The results showed that the antibacterial protein SBL200 could exert antibacterial activity in the milk environment. These results showed that the antibacterial protein SBL200 could be used as an active ingredient in treating bovine mastitis.

### Example 5: Investigation of Treatment Effects on Bovine Mastitis

Therapeutic effects of the antibacterial protein SBL200 specific for *Streptococci* on bovine mastitis were investigated. Specifically, whether a composition containing the antibacterial protein SBL200 prepared in Example 2 of the present disclosure was effective in treating bovine mastitis was investigated on 6 dairy cows with bovine mastitis caused by *Streptococci.* The dairy cows were divided into two groups of 3 each. The treatment group was administered a composition containing the antibacterial protein SBL200 daily through the nipple. The control group was administered the same composition without the antibacterial protein SBL200 daily through the nipple. The injection volume was 5 mL per time. While this treatment was performed for 10 days, the number of somatic cells contained in the raw milk collected from the dairy cows before and after the 10-day treatment was tested according to a conventional method. When bovine mastitis occurred, white blood cells increased to prevent the invasion of pathogens. At this time, the white blood cells that died through the fight against pathogens were called somatic cells. Types of the somatic cells included mammary epithelial cells, immune cells (white blood cells), neutrophils, lymphocytes, and monocytes. In the experiment, direct microscopy, which was the easiest to perform, was used as a somatic cell examination method. To explain this briefly, raw milk samples were spread, dried, and stained within 1 cm² of a slide glass. Thereafter, somatic cell counts were examined directly under a microscope and multiplied by the microscope count. In this way, the number of somatic cells in 1 mL of raw milk was calculated. The results are as follows. The results in Table 4 below are the average values of 3 animals in each experimental group. Since the values do not deviate significantly from the average, the provision of standard deviation is omitted.

**[Table 4]**

| Treatment Effects of Bovine Mastitis | | |
|---|---|---|
| Division | Somatic Cell Count per mL of Raw Milk (CFU/mL) | |
| | Treatment Group | Control Group |
| Before Treatment | 5.2× 10⁴ | 5.0× 10⁴ |
| After Treatment | 1.6× 10³ | 7.8× 10⁴ |

In the table above, the contents corresponding to the treatment group are the results from the group administered the composition containing the antibacterial protein SBL200, meanwhile, the contents corresponding to the control group are the results from the group administered the composition, which did not contain the antibacterial protein SBL200 and had the same remaining composition.

As can be seen from the results, it was confirmed that there was a significant bovine mastitis treatment effect only when the composition containing the antibacterial protein SBL200 of the present disclosure was administered. From these results, it was confirmed that the composition containing the antibacterial protein SBL200 of the present disclosure as an active ingredient was effective in treating bovine mastitis. These properties show that the composition containing the antibacterial protein SBL200 of the present disclosure as an active ingredient could be used for the purpose of treating bovine mastitis.

The specific parts of the present disclosure have been described in detail above. For those skilled in the art, these specific techniques are merely examples of preferred implementations. It is clear that the scope of the present disclosure is not limited by these examples. Accordingly, the actual scope of the present disclosure will be defined by the appended claims and their equivalents.
Depository Institution Name: KCTC
Accession Number: KCTC15032BP
Deposit Date: 20220712

### BUDAPEST TREATY ON THE INTERNATIONAL RECOGNITION OF THE DEPOSIT OF MICROORGANISMS FOR THE PURPOSE OF PATENT PROCEDURE INTERNATIONAL FORM

### RECEIPT IN THE CASE OF AN ORIGINAL DEPOSIT

### Issued pursuant to Rule 7.1

To: iNtRON Biotechnology
Address: 137, Sagimakgol-ro, Jungwon-gu, Seongnam-si, Gyeonggi-do, Republic of Korea

| **I.** IDENTIFICATION OF THE MICROORGANISM | |
|---|---|
| Identification reference given by the DEPOSITOR: ***Escherichia coli* M-2** | Accession number given by the INTERNATIONAL DEPOSITARY AUTHORITY: **KCTC 15032BP** |

| **II.** SCIENTIFIC DESCRIPTION AND/OR PROPOSED TAXONOMIC DESIGNATION | |
|---|---|
| The microorganism identified under I above was accompanied by: | |
| [ ] a scientific description | |
| [ ] a proposed taxonomic designation (Mark with a cross where applicable) | |

| **III.** RECEIPT AND ACCEPTANCE | |
|---|---|
| This International Depositary Authority accepts the microorganism identified under I above, which was received by it on **July 12,** 2022. | |

| **IV.** RECEIPT OF REQUEST FOR CONVERSION | |
|---|---|
| This microorganism identified under **I** above was received by the International Depositary Authority on and a request to convert the original deposit to a deposit under the Budapest Treaty was received by it on. | |

| **V.** INTERNATIONAL DEPOSITARY AUTHORITY | |
|---|---|
| Name: Korean Collection for Type Cultures | Signature(s) of person(s) having the power to represent the International Depositary |
| Address: 181, Ipsin-gil, Jeongeup-si, Jeonbuk-do, Republic of Korea (56212) | Authority or of authorized official(s): |
| | Representative |
| | Date: **July 12, 2022** |

## Claims

1. An antibacterial protein SBL200 with an amino acid sequence represented by SEQ ID NO: 1, which has streptococci-specific antibacterial activity.

2. The antibacterial protein SBL200 of Claim 1, wherein the antibacterial protein SBL200 is made of 291 amino acids and has a molecular weight of 32.5 kDa.

3. A composition for treating infections caused by *Streptococci,* the composition comprising the antibacterial protein SBL200 of claim 1 as an active ingredient.

4. The composition of claim 3, wherein the composition is used for treating bovine mastitis.

5. A method for preparing the antibacterial protein SBL200 of claim 1 by using a production strain *Escherichia coli* M-2 (accession number KCTC15032BP) constructed by transforming *E. coli* with a plasmid containing a nucleic acid sequence represented by SEQ ID NO: 2.
